**Europäisches Patentamt**

**European Patent Office**

**Office européen des brevets**

(11) Veröffentlichungsnummer: **0 197 360 B1**

## EUROPÄISCHE PATENTSCHRIFT

(45) Veröffentlichungstag der Patentschrift: **21.04.93**

(51) Int. Cl.⁵: **A23K 1/17**, C12P 1/04

(21) Anmeldenummer: **86103539.2**

(22) Anmeldetag: **15.03.86**

(54) **Verwendung von Efomycinen als Leistungsförderer bei Tieren, Efomycine und ihre Herstellung.**

(30) Priorität: **30.03.85 DE 3511753**

(43) Veröffentlichungstag der Anmeldung:
**15.10.86 Patentblatt 86/42**

(45) Bekanntmachung des Hinweises auf die
Patenterteilung:
**21.04.93 Patentblatt 93/16**

(84) Benannte Vertragsstaaten:
**AT BE CH DE FR GB IT LI NL SE**

(56) Entgegenhaltungen:
**US-A- 3 076 746**

**CHEMICAL ABSTRACTS, Band 104, Nr. 2, 13.
Januar 1986, Seite 321, Spalte 1, Zusammenfassung Nr. 10615j, Columbus, Ohio, US; &
JP A - 60 152 418 (TOKYO TANABE CO.)
10.08.85 (Kat. P,A)**

**J. of Anim. Sci (45), S. 385-392 (1977)**

(73) Patentinhaber: **BAYER AG**

**W-5090 Leverkusen 1 Bayerwerk(DE)**

(72) Erfinder: **Frobel, Klaus, Dr.
Birkenhöhe 5
W-5600 Wuppertal 1(DE)**
Erfinder: **Bischoff, Erwin, Dr.
Pahlkestrasse 73
W-5600 Wuppertal 1(DE)**
Erfinder: **Müller, Hartwig, Dr.
Steinstrasse 15
W-5620 Velbert(DE)**
Erfinder: **Salcher, Olga, Dr.
Am Eckbusch 43/110
W-5600 Wuppertal 1(DE)**
Erfinder: **De Jong, Anno, Dr.
Stockmannsmühle 46
W-5600 Wuppertal 1(DE)**
Erfinder: **Berschauer, Friedrich, Dr.
Claudiusweg 9
W-5600 Wuppertal 1(DE)**
Erfinder: **Scheer, Martin, Dr.
Herberts-Katernberg 7
W-5600 Wuppertal 1(DE)**

**Beschreibung**

Die vorliegende Erfindung betrifft die Verwendung mikrobiologisch hergestellter Verbindungen und ihrer Gemische, die Efomycine genannt werden, als Leistungsförderer bei Tieren sowie Verfahren zu ihrer Herstellung, ferner neue Efomycine sowie ihre Gemische als neue chemische Verbindungen. Die Erfindung beschreibt ferner die Herstellung der Efomycine und ihrer Gemische sowie verwendbare Mikroorganismen.

1. Es wurde gefunden, daß man die Efomycine A-F sowie ihre Gemische als Leistungsförderer bei Tieren einsetzen kann.

Zusätzlich zeigen die Efomycine A-F und ihre Gemische starke antimikrobielle wie z.B. antibakterielle und antivirale Wirkungen.

2. Es wurden ferner die neuen Efomycine A, B, C, D, F mit den im folgenden angegebenen chemischen und physikalischen Eigenschaften sowie Gemische dieser Verbindungen gefunden:

2.1 Die neue Verbindung Efomycin A wird durch die folgenden chemischen und physikalischen Eigenschaften charakterisiert:

1.

| Die Elementaranalyse | C: | 61,3-63,8 |
|---|---|---|
| | H: | 8,2- 9,4 |
| | O: | 26,8-30,5 |

Es muß hier darauf hingewiesen werden, daß bei höhermolekularen Naturstoffen die Fehlerbreite der Elementaranalyse größer sein kann als allgemein üblich (R.B. Woodward, Angew. Chem. $\underline{69}$, 50-51 (1957)).

2. Die Summenformel: $C_{55}H_{92}O_{18}$

3. Das Massenspektrum (Fast Atom Bombardment) Molmasse + $Na^+$ : 1061

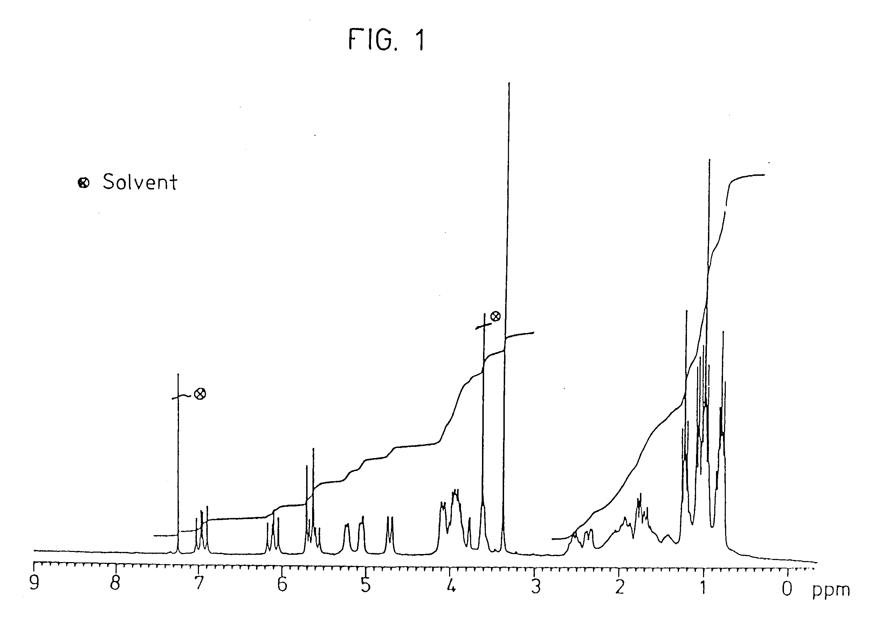

4. Das $^1$H-Kernresonanzspektrum, angegeben in Teilen pro Million, gemäß Abb. 1

Es wurde in einem WP 200 der Fa. Bruker bei 200 MHz an einer Lösung von Efomycin A in deuteriertem Chloroform mit Tetramethylsilan als innerem Standard aufgenommen.

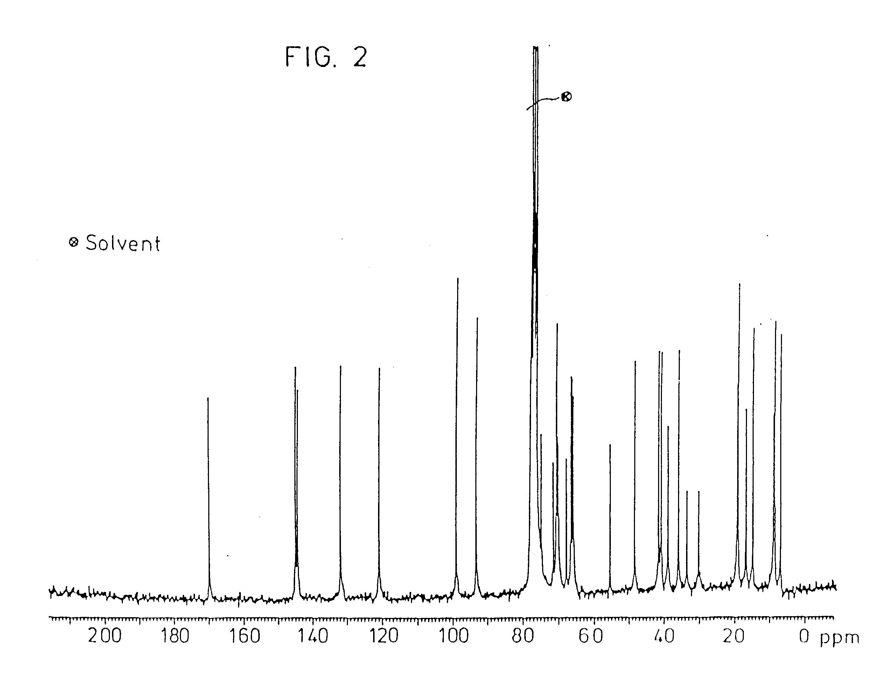

5. Das $^{13}$C-Kernresonanzspektrum, angegeben in Teilen pro Million, gemäß Abb. 2

Es wurde in einem EP 200 der Fa. Bruker bei 50,32 MHz an einer Lösung von Efomycin A in deuteriertem Chloroform mit Tetramethylsilan als innerem Standard aufgenommen.

6. Das UV-Absorptionsmaximum bei 251-254 nm in methanolischer Lösung.

2.2 Die neue Verbindung Efomycin B wird durch die folgenden chemischen und physikalischen Eigenschaften charakterisiert:

1.

| Die Elementaranalyse | C: | 61,3-63,8 |
|---|---|---|
| | H: | 8,2- 9,4 |
| | O: | 26,8-30,5 |

Es muß hier darauf hingewiesen werden, daß bei höhermolekularen Naturstoffen die Fehlerbreite der Elementaranalyse größer sein kann als allgemein üblich (R.B. Woodward, Angew. Chem. $\underline{69}$, 50-51 (1957)).

2. Die Summenformel $C_{56}H_{94}O_{18}$ 7)). Das Massenspektrum (Fast Atom Bombardment)

3. Molmasse + $Na^+$ : 1075

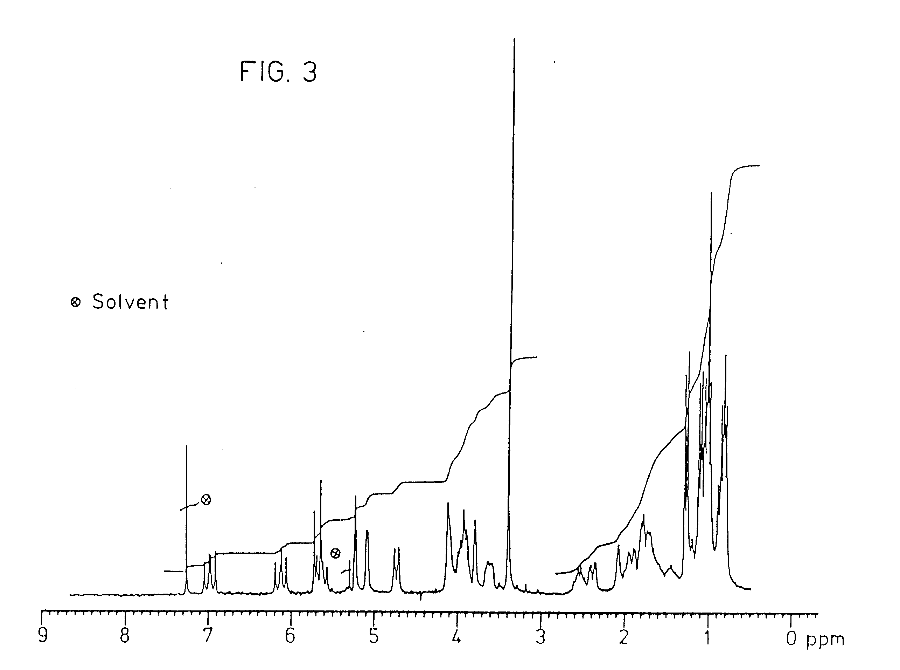

4. Das $^1$H-Kernresonanzspektrum, angegeben in Teilen pro Million, gemäß Abb. 3.

Es wurde in einem EP 200 der Fa. Bruker bei 200 MHz an einer Lösung von Efomycin B in deuteriertem Chloroform mit Tetramethylsilan als innerem Standard aufgenommen.

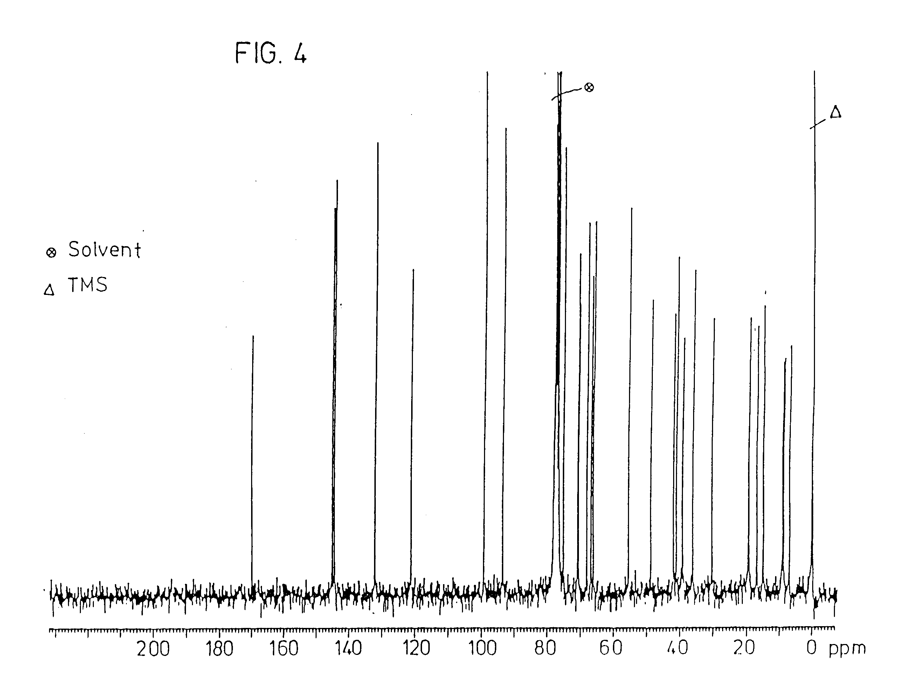

5. Das $^{13}$C-Kernresonanzspektrum, angegeben in Teilen pro Million, gemäß Abb. 4.

Es wurde in einem EP 200 der Fa. Bruker bei 50,32 MHz an einer Lösung von Efomycin A in deuteriertem Chloroform mit Tetramethylasilan als innerem Standard aufgenommen.

6. Das UV-Absorptionsmaximum bei 251-254 nm in methanolischer Lösung.

2.3 Die neue Verbindung Efomycin C wird durch die folgenden chemischen und physikalischen Eigenschaften charakterisiert.

1. Die Summenformel $C_{49}H_{80}O_{15}$

2. Das Massenspektrum (Fast Atom Bombardment) Molmasse + $Na^+$: 931

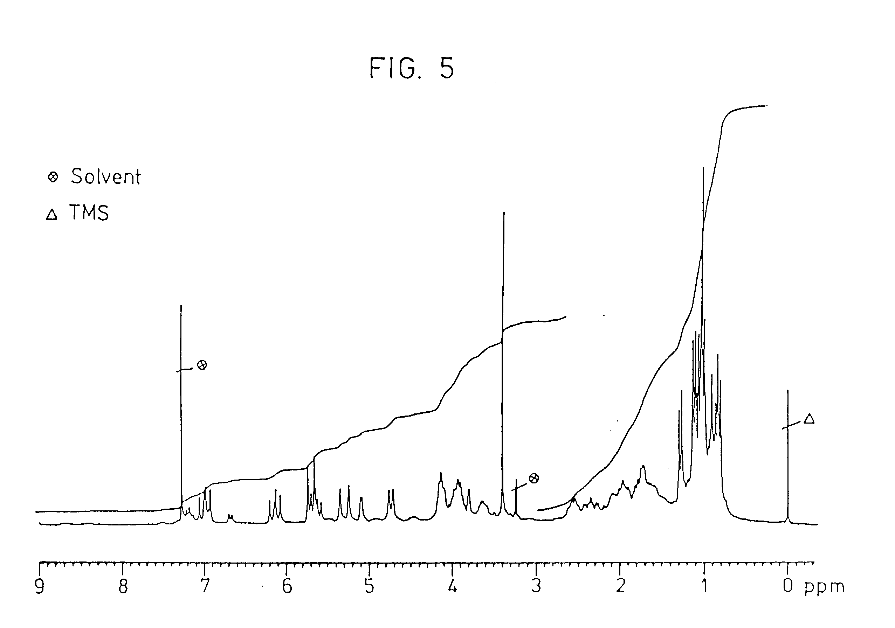

3. Das $^1$H-Kernresonanzspektrum, angegeben in Teilen pro Million, gemäß Abb. 5 Es wurde in einem WP 200 der Fa. Bruker bei 200 MHz an einer Lösung von Efomycin C in deuteriertem Chloroform mit Tetramethylsilan als innerem Standard aufgenommen.

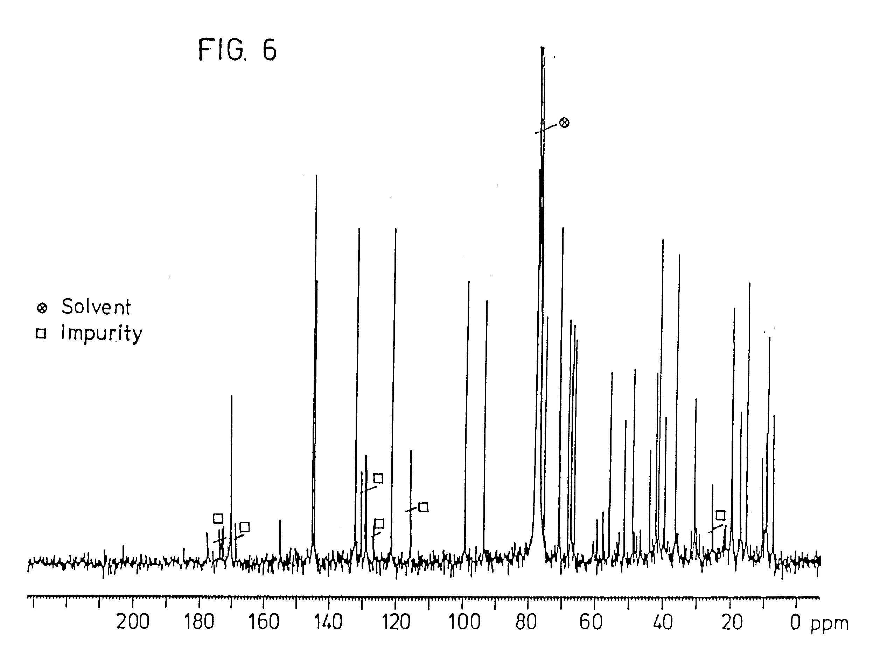

4. Das $^{13}$C-Kernresonanzspektrum, angegeben in Teilen pro Million, gemäß Abb. 6.

Es wurde in einem WM 250 der Fa. Bruker bei 250 MHz an einer Lösung von Efomycin C in deuterisiertem Chloroform mit Tetramethylsilan als innerem Standard aufgenommen.

5. Das UV-Absorptionsmaximum bei 251-254 nm in methanolischer Lösung.

2.4 Die neue Verbindung Efomycin D wird durch die folgenden chemischen und physikalischen Eigenschaften charakterisiert

1. Die Summenformel $C_{42}H_{68}O_{12}$

2. Das Massenspektrum (Fast Atom Bombardment) Molmasse + $Na^+$: 787

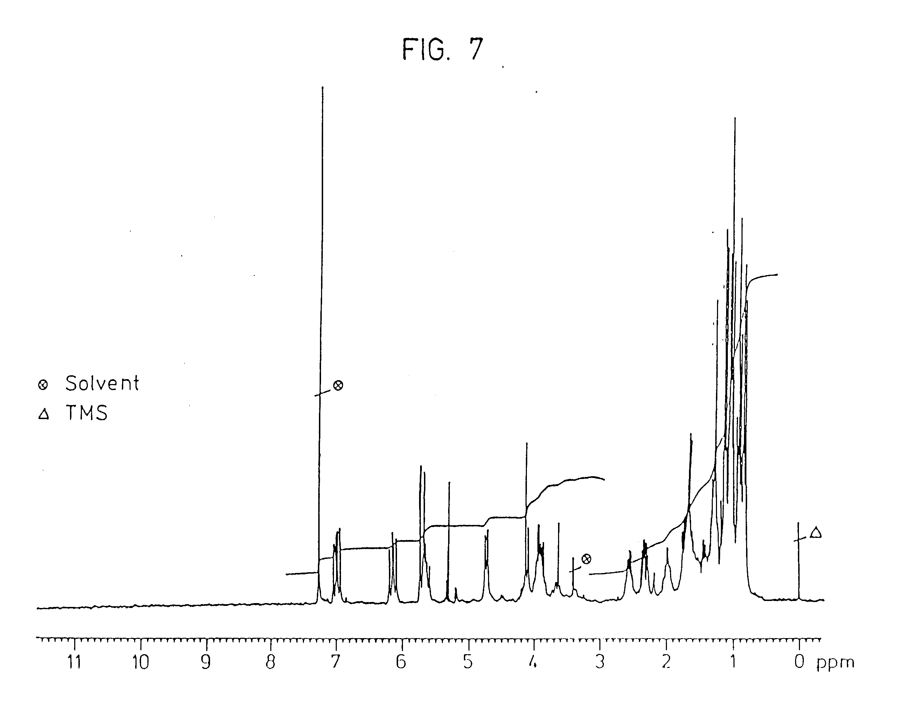

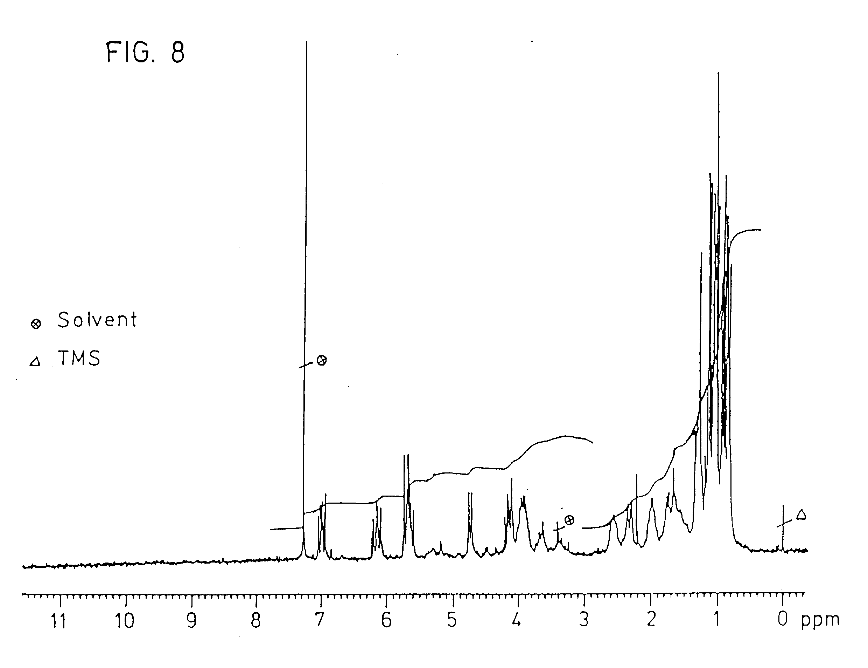

3. Die $^1$H-Kernresonanzspektren, angegeben in Teilen pro Million. Sie werden in einem WM 250 der Fa. Bruker bei 250 MHz an einer Lösung von Efomycin D in deuteriertem Chloroform mit Tetramethylsilan als innerem Standard aufgenommen (Abb. 7). Die identische Lösung wurde nach Schütteln mit deuteriertem Wasser unter entsprechenden Bedingungen aufgenommen (Abb. 8).

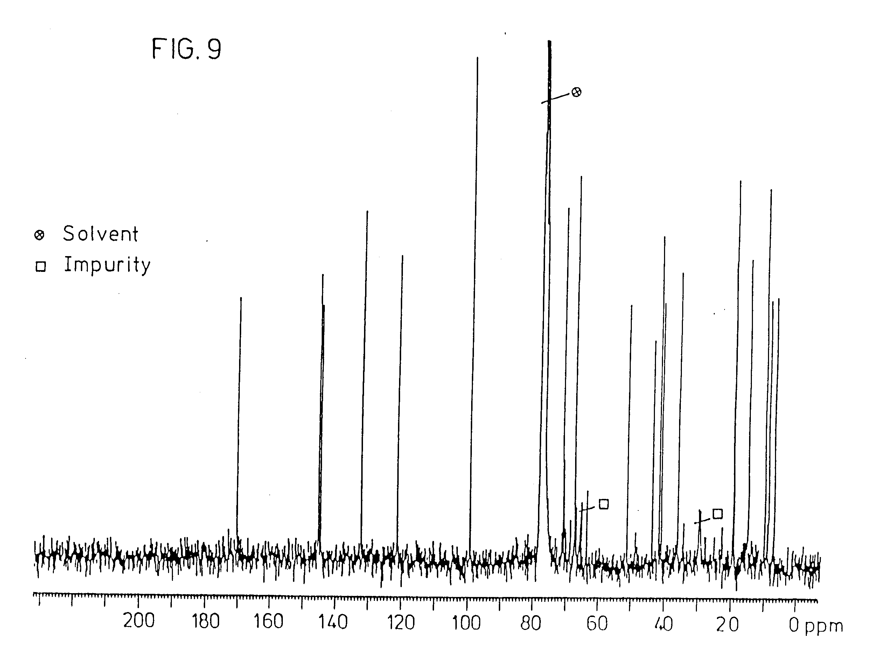

4. Das $^{13}$C-Kernresonanzspektrum, angegeben in Teilen pro Million, gemäß Abb. 9.

Es wurde in einem WM 250 der Fa Bruker bei 62,84 MHz an einer Lösung von Efomycin D in deuteriertem Chloroform mit Tetramethylsilan als innerem Standard aufgenommen.

5. Das UV-Absorptionsmaximum bei 251-254 nm in methanolischer Lösung.

2.5 Die neue Verbindung Efomycin F wird durch die folgenden chemischen und physikalischen Eigenschaften charakterisiert:

1. Die Summenformel: $C_{48}H_{78}O_{15}$

2.

| Die Elementaranalyse: | C: | 63,0-65,6 |
|---|---|---|
| | H: | 8,5- 8,9 |
| | O: | 26,5-28,5 |

Es muß hier daraufhingewiesen werden, daß bei höhermolekularen Naturstoffen die Fehlerbreite der Elementaranalyse größer sein kann als allgemein üblich (R.B. Woodward, Angew. Chem. 69, 50-51 (1957)).

3. Das Massenspektrum (Fast Atom Bombardment) Molmasse + $Na^+$: 917

4. Das UV-Absorptionsmaximum bei 251-254 nm in methanolischer Lösung.

5. Die folgenden Absorptionsbanden, angegeben in $cm^{-1}$, des IR-Spektrums, aufgenommen als KBr Preßling:

| 3444 | 1385 | 1150 | 745 |
|---|---|---|---|
| 2975 | 1304 | 1112 | |
| 2937 | 1284 | 1088 | |
| 1693 | 1259 | 1000 | |
| 1642 | 1226 | 880 | |
| 1462 | 1187 | 825 | |

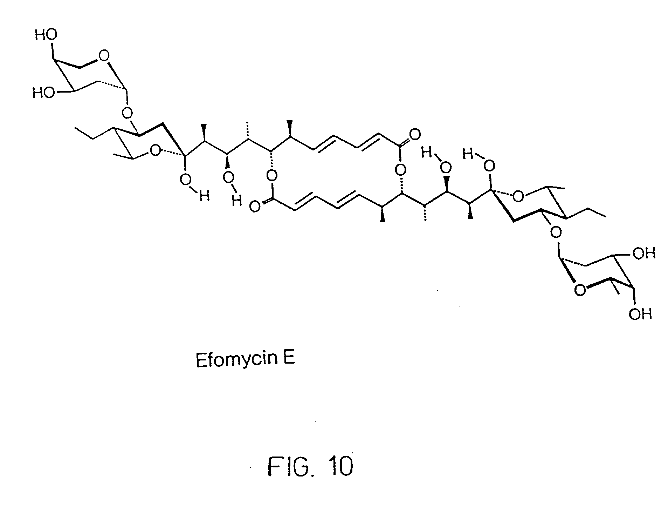

Die Verbindung Efomycin E wird durch die in Abb. 10 wiedergegebene Strukturformel gekennzeichnet.

Efomycin E ist mit Azalomycin B (vgl. US-P 3 076 746) bzw. Elaiophylin (W. Keller-Schierlein et.al. Hel.Chim.Acta 64, 407-424 (1981) ; K. Neupert-Laves, M. Dobler Helv. Chim. Acta 65, 262-267 (1982)) in Konstitution, sowie relativer und absoluter Konfiguation sämtlicher, bei Raumtemperatur in Lösung konfigurativ stabiler, asymmetrischer Kohlenstoffatome identisch.

3. Es wurde weiterhin gefunden, daß man die erfindungsgemäßen Efomycine A, B, C, D, F sowie ihre Gemische erhält, wenn man geeignete Mikroorganismen der Familie der Streptomycetaceae in einem Nährmedium welches assimilierbare Kohlenstoff- und Stickstoffquellen sowie Mineralsalze enthält unter aeroben Bedingungen kultiviert, das entstandene Gemisch der Efomycine nach üblichen Methoden isoliert und trennt.

Bei Kenntnis der Eigenschaften der Efomycine A, B, C, D, F ist es mit Hilfe der üblichen chromatografischen, spektroskopischen und/oder biologischen Nachweisverfahren leicht und rasch möglich, geeignete

EP 0 197 360 B1

Mikroorganismenstämme auszumachen die Efomycine A, B, C, D, F produzieren.

4. Zur Durchführung des Verfahrens kann insbesondere der Streptomyces Stamm BS 1261 - sowie seine Mutanten und Varianten - Verwendung finden.

Dieser Stamm gehört zu der Familie Streptomycetaceae, der Gattung Streptomyces aus der grauen Serie der Streptomyceten (Cinereus-Gruppe).

Der Stamm BS 1261 wurde am 16.01.1985 bei der Deutschen Sammlung für Mikroorganismen (DSM) Griesbachstraße 8, 3400 Göttingen, Bundesrepublik Deutschland unter der Nummer DSM 3200 hinterlegt.

Taxonomische Beschreibung des Stamms BS 1261 (DSM 3200)

Die taxonomische Beschreibung des Stamms BS 1261 erfolgte nach Bergey's Manual of Determinative Bacteriology 8th, (1974) sowie International Journal of Systematic Baceteriology 16, 313-340 (1966) und The Prokaryotes 2., 2028-2020 (1981).

1. Morphologie

Eine gute Sporulation war auf den ISP-Medien Nr. 2, 3, 4, 5, 7 bis 9 zu beobachten. Auf ISP-Medium Nr. 9 mit Ribose als C-Quelle sowie aus ISP-Medium Nr. 1 und 6 wurde überwiegend Substratmyzel gebildet.

Luftmyzel (ISP-Medium Nr. 3, 28°C, 7 Tage:

Farbe: grau (Cinereus-Typ)
Sporenketten: Retinaculum-Apertum-Type
Sporen: Quadratisch bis rechteckig, 1,4-1,8 $\mu$m lang und 1,3-1,6 $\mu$m breit
glatt (Elektronenmikroskopie).

Substratmyzel:

Farbe: braun

2. Angaben zur Physiologie

Das Temperaturoptimum liegt bei 28°C (auf ISP-Medium Nr. 2, 5 Tage). Der Stamm wächst nicht bei 4° und 45°C. Melanin wird nicht gebildet. Das Wachstum wird durch die Antibiotika Erythromycin (10 $\mu$g), Sulphafurazol (100 $\mu$g), Streptomycin (10 $\mu$g) und Novobiocin (5 $\mu$g) gehemmt (ISP-Medium Nr. 2, 28°C, 2 Tage).

Die Verwertung von C-Quellen wurde auf Basalagar (ISP-Medium Nr. 9) nach Int. Syst. Bact. 16, 313-340 (1966) überprüft. Für die Negativ-Kontrolle wurde das Wachstum auf Basalagar ohne C-Quelle verglichen. Dabei werden folgende Ergebnisse erhalten:

4

Tabelle

Verwertung von C-Quellen durch Stamm BS 1261

| C-Quelle (10 g/l) | Wachstum [*] |
|---|:---:|
| Kontrolle (keine C-Quelle) | - |
| D-Glucose | + |
| L-Arabinose | + |
| L-Rhamnose | + |
| B-Fructose | + |
| L-Galactose | + |
| Raffinose | + |
| D-Mannit | + |
| meso-Inosit | + |
| Salicin | + |
| Saccharose | + |
| Ribose | + |
| Mannose | + |
| Maltose | + |
| Mellibiose | + |
| Cellulose | - |
| Acetat | - |

)[*]  + = Wachstum, - = kein Wachstum.

3. Besonders geeignetes Medium für Wachstum und Sporulation

ISP 3 (Hafermehl-Agar)

20 g Hafermehl werden in 1000 ml $H_2O$ deion. suspendiert und 20 Minuten gekocht. Anschließend wird filtriert, 1 ml Spurenelementlösung für ISP3 und 18 g Agar zugegeben, und der pH-Wert auf 7,2 eingestellt, und bei 121 ° C 15-20 Minuten autoklaviert.

Spurenelementlösung für ISP 3

| | |
|---|---|
| $FeSO_4$ 7 $H_2O$ | 0,1 g |
| $MnCl_2$ . 4 $H_2O$ | 0,1 g |
| $ZnSo_4$ . 7 $H_2O$ | 0,1 g |
| $H_2O$ deion. | 100 ml |

Weitere Medien s. Int. J. Syst. Bact. 16, 313-340 (1966).

Der Stamm BS 1261, isoliert aus einer Erdprobe aus Neuseeland, läßt sich aufgrund der morphologischen Daten in die graue Serie der Streptomyceten (Cinereus-Gruppe) einordnen.

Taxonomische Bezeichnung: Streptomyces sp.

Die Efomycine A-F werden durch die Fermentation geeigneter Mikroorganismen, wie dem Streptomycetenstamm BS 1261 oder dessen Mutanten oder Varianten erzeugt.

Das Fermentationsverfahren kann mit Hilfe fester, halbfester oder flüssiger Nährmedien durchgeführt werden. Bevorzugt werden wäßrig-flüssige Nährmedien verwendet.

Die Beimpfung der Nährmedien erfolgt nach allgemein üblichen Methoden, z.B. über Schrägröhrchen oder Kolbenkulturen.

Die Kultur erfolgt unter aeroben Bedingungen und kann gemäß den allgemein üblichen Methoden wie unter Verwendung von Schüttelkulturen oder von Submerskulturen durchgeführt werden. Bevorzugt erfolgt die Kultivierung im aeroben Submersverfahren in belüfteten Fermentern, z.B. in üblichen Submersfermentationstanks. Es ist möglich, die Fermentation kontinuierlich oder diskontinuierlich durchzuführen. Vorzugsweise wird diskontinuierlich gearbeitet.

Die Kultur wird in Nährmedien durchgeführt, welche bekannterweise zur Kultivierung von Mikroorganismen der Ordnung Actinomycetales verwendet werden. Das Nährmedium muß eine oder mehrere assimilierbare Kohlenstoffquellen und Stickstoffquellen sowie Mineralsalze enthalten, wobei diese Produkte in Form von definierten Einzelbestandteilen, aber auch in Form von komplexen Gemischen, wie sie insbesondere biologische Produkte verschiedenen Ursprungs darstellen, vorliegen können. Als Kohlenstoffquellen kommen alle üblichen Kohlenstoffquellen infrage. Beispielsweise seien Kohlenhydrate, insbesondere Polysaccharide, wie Stärke oder Dextrine, Disaccharide, wie Maltose oder Lactose, Monosaccharide, wie Glucose oder Xylose, Alkohole, wie Mannit oder Glycerin sowie natürlich vorkommende Gemische, wie Malzextrakt, Melasse oder Molkepulver genannt. Als Stickstoffquellen kommen alle üblichen organischen und anorganischen Stickstoffquellen infrage. Beispielsweise seien Eiweißstoffe, Eiweißhydrolysate, Aminosäuren, Nucleosidbasen, wie Cytosin oder Uracil sowie Sojabohnenmehl, Baumwollsamenmehl, Linsenmehl, Erbsenmehl, lösliche und unlösliche pflanzliche Proteine, Maisquellwasser, Hefeextrakt, Peptone und Fleischextrakt, stickstoffhaltige Salze wie z.B. Ammoniumsalze und Nitrate, aufgeführt. Die Mineralsalze, welche im Nährmedium enthalten sein sollten, liefern z.B. folgende Ionen:
$Mg^{++}$, $Na^+$, $K^+$, $Ca^{++}$, $NH_4^+$, $Cl^-$, $SO_4^{--}$, $PO_4^{---}$ und $NO_3^-$
sowie Ionen der üblichen Spurenelemente, wie Cu, Fe, Mn, Mo, Zn, CO, Ni. Falls die Kohlenstoff- oder Stickstoffquellen bzw. das verwendete Wasser nicht ausreichend diese Salze bzw. Spurenelemente enthalten, ist es zweckmäßig, das Nährmedium entsprechend zu ergänzen. Die Zusammensetzung der Nährmedien kann in weiten Bereichen variiert werden. Art und Zusammensetzung der Nährmedien werden im allgemeinen davon abhängig sein, welche Bestandteile jeweils besonders günstig zur Verfügung stehen. Im allgemeinen enthalten die Nährlösungen vorzugsweise etwa 0,5 bis 8 %, insbesondere 0,6 bis 6 % Kohlenstoffquellen, vorzugsweise etwa 0,5 bis 4 %, insbesondere 0,5 bis 2 % Stickstoffquellen und vorzugsweise etwa 0,001 bis 0,5 %, insbesondere 0,003 bis 0,3 % Mineralsalze.

Bei der Durchführung des Verfahrens kann es günstig sein, zu Beginn der Kultivierung nur relativ geringe Konzentrationen der löslichen Nährlösungsbestandteile zu verwenden und dann im Laufe der ersten 3 Kultivierungstage durch häufigere Zusätze diese Bestandteile in Form steriler, relativ konzentrierter Lösungen dem Kulturansatz fraktioniert zuzufüttern.

Der pH-Wert der wachsenden Kulturen sollte vorzugsweise zwischen etwa 5 und etwa 10, insbesondere zwischen 6,5 und 8,0 gehalten werden. Ein zu starker pH-Abfall in den sauren Bereichen kann durch Zusätze einer organischen oder anorganischen Base, vorzugsweise von $CaCO_3$ vermieden werden. Wie in der Fermentationstechnologie üblich, kann auch eine automatische pH-Regulierung durchgeführt werden, bei der sterile organische oder anorganische Säuren, z.B. $H_2SO_4$, oder sterile Laugen, z.B. NaOH in Abständen in die Kulturlösung eingespritzt werden.

Es ist zweckmäßig sicherzustellen, daß die Mikroorganismen ausreichend mit Sauerstoff sowie den Nährstoffen in Kontakt gebracht werden. Dies kann nach den allgemein üblichen Methoden wie Schütteln und Rühren erfolgen.

Die Züchtungstemperatur kann zwischen etwa 24°C und etwa 34°C, vorzugsweise zwischen 26°C und 32°C liegen, besonders bevorzugt liegt sie bei etwa 28°C. Die Dauer der Züchtung kann stark variiert werden, wobei z.B. die Zusammensetzung des Nährmediums und die Züchtungstemperatur eine Rolle spielen. Die jeweiligen optimalen Bedingungen können von jedem Fachmann auf dem mikrobiologischen Gebiet leicht festgelegt werden.

Es hat sich herausgestellt, daß die Menge der sich in der Kulturbrühe anreichernden erfindungsgemäßen Verbindungen im allgemeinen ihr Maximum etwa 1 bis 10, vorzugsweise etwa 4 bis 7 Tage nach Züchtungsbeginn erreicht. Das gewünschte Endprodukt der Fermentation kann mit Hilfe von dünnschichtchromatographischen und hochdruckflüssigkeitschromatographischen Untersuchungen oder biologischen Testverfahren bestimmt werden.

Wie allgemein bei mikrobiologischen Verfahren üblich sollten Fremdinfektionen der Kulturmedien vermieden werden. Hierzu werden die üblichen Vorkehrungen getroffen, wie Sterilisation der Nährmedien, Kulturgefäße sowie der für die Belüftung notwendigen Luft. Zur Sterilisation der Vorrichtungen können z.B. die Dampfals auch die Trockensterilisation verwendet werden, wobei die Temperaturen vorzugsweise bei 100 bis 140°C, insbesondere bei 120 bis 130°C liegen können.

Falls bei der Kultivierung in unerwünschter Menge Schaum entsteht, können die üblichen chemischen Schaumdämpfungsmittel, z.B. flüssige Fette und Öle, wie Öl-Wasser-Emulsionen, Paraffine, höhere Alkohole, wie Octadecanol, Siliconöle, Polyoxyethylen- bzw. Polyoxypropylenverbindungen (z.B. in Mengen bis etwa 1 %) zugesetzt werden. Schaum kann auch mit Hilfe der üblichen mechanischen Vorrichtungen (welche z.B. Zentrifugalkräfte benutzen) gedämpft oder beseitigt werden.

Die erfindungsgemäßen Verbindungen können aus dem Kulturmedium nach allgemein üblichen physikalisch-chemischen Methoden isoliert werden. Die Isolierung kann z.B. gemäß den üblichen Extraktionsverfahren, Fällungsverfahren und/oder Chromatographieverfahren erfolgen. Die isolierten Stoffe können mit Hilfe der genannten Methoden auch feingereinigt werden. Für viele Fälle ist jedoch eine Feinreinigung nicht erforderlich, da gegebenenfalls vorhandene geringfügige Verunreinigungen die Wirksamkeit der Verbindungen nicht nachteilig beeinflußen. Bei allen Isolierungs- und Reinigungsoperationen ist darauf zu achten, daß die pH-Werte im neutralen Bereich liegen. Vorzugsweise werden pH-Werte zwischen 7 und 8 eingehalten. Zur Einstellung des pH-Wertes können anorganische und organische Basen verwendet werden, wie Alkalibasen z.B. NaOH, KOH oder organische Amine, wie Triethylamin; anorganische Säuren wie z.B. HCl und organische Säuren wie z.B. Essigsäure.

Um bei den oben angegebenen Isolierungs- und Reinigungsmethoden die Fraktionen herauszufinden, in welchen die erfindungsgemäßen Verbindungen in höchster Konzentration bzw. Reinheit vorliegen, können die üblichen physikalisch chemischen Methoden z.B. Messen einer charakteristischen Bande im Spektrum oder der $R_f$-Werte, Bestimmung der antibakteriellen Aktivität usw. herangezogen werden. Diese Methoden können auch verwendet werden, um in Routineverfahren für die Produktion von Efomycinen geeignete Mikroorganismen aufzufinden.

Die Isolierung und Reinigung der erfindungsgemäßen Verbindungen kann z.B. im Falle, daß ein flüssiges wäßriges Nährmedium verwendet wird, wie folgt vorgenommen werden:

Da die Efomycine sowohl im Kulturüberstand als auch im Myzel zu finden sind, können sie mit Hilfe von üblichen Extraktionsverfahren, Fällungsverfahren und/oder Chromatographieverfahren aus dem Fermentationsansatz isoliert und gegebenenfalls gereinigt werden. Die Chromatographie kann in Form der Säulenchromatographie durchgeführt werden. Mit gutem Erfolg läßt sich auch die Hochdruckflüssigkeitschromatographie (HPLC) einsetzen. Als Adsorptionsmittel können die üblichen anorganischen oder organischen Adsorptionsmittel eingesetzt werden, wie z.B. Kieselgel, Magnesiumsilikat, Aktivkohle, Cellulose, Cellulosederivate, Kunstharze wie Polyamide, z.B. acetyliertes Polyamid, Dextrangele oder modifizierte Dextrangele. Als Laufmittel können die verschiedensten Lösungsmittel oder Lösungsmittelgemisch verwendet werden, in welchen die erfindungsgemäßen Verbindungen löslich sind. Bevorzugt werden Essigsäureethylester, Chloroform und Methanol oder ihre Gemische (beispielsweise Gemische aus Chloroform und Methanol oder aus Essigsäureethylester und Chloroform) eingesetzt.

Bevorzugt werden zur Isolierung der erfindungsgemäßen Verbindungen Chromatographie-Verfahren verwendet, z.B. unspezifische Adsorption an Sorbentien wie Kieselgel oder andererseits Geldiffusionschromatographie. Dies sind die von der Reinigung schlecht wasserlöslicher Naturstoffe her bekannten Methoden.

Aus ihren Lösungen können die erfindungsgemäßen Verbindungen nach den üblichen Methoden, z.B. Verdampfen des Lösungsmittels, Gefriertrocknung usw. erhalten werden.

In einer bevorzugten Ausführungsform wird das Mycelium aus der Kulturbrühe, vorzugsweise durch Zentrifugation abgetrennt und mit einem, mit Wasser mischbaren Lösungsmittel mehrfach, vorzugsweise zweimal extrahiert. Als Lösungsmittel können ($C_1$-$C_4$)-Alkylalkohole, $C_{1-4}$-Ketone, besonders bevorzugt Aceton verwendet werden. Die wäßrig-organische Lösung wird im Vakuum, z.B. etwa auf 1/20 des Volumens der Kulturbrühe konzentriert und gefriergetrocknet.

Dieses Rohpräparat wird in Wasser suspendiert und die Efomycine mit einem mit Wasser nicht mischbaren Lösungsmittel, wie z.B. Chlorkohlenwasserstoffe wie Chloroform, Ester der Essigsäure oder Ketone extrahiert. Aus diesem Extrakt können die Efomycine durch übliche chromatographische Methoden, vorzugsweise Chromatographie an Kieselgel, isoliert werden.

Die Efomycine können auch aus dem Kulturfiltrat durch Extraktion mit einem mit Wasser nicht mischbaren Lösungsmittel wie z.B. Essigester, Methylenchlorid oder Chloroform extrahiert werden.

Sie können außerdem an unspezifische Adsorberharze auf Polystyrolbasis (z.B. Amberlite XAD der Firma Roehm & Hass oder Lewatit OC 1031 der Firma Bayer) gebunden werden. Die Desorption wird fraktioniert, durch Mischungen aus Wasser und organischen Lösungsmitteln, insbesondere Wasser/Methanol vorgenommen. Die durch Test gegen Staphylococcus aureus 1756 ermittelten aktiven Fraktionen werden unter reduziertem Druck bis zur vollständigen Entfernung des organischen Lösungsmittels bei 30 - 35°C konzentriert und der Rückstand in ca. 1/100 des Volumens des Kulturfiltrates suspendiert und gefriergetrocknet.

Das Lyophilisat wird erneut in Wasser suspendiert und vorzugsweise mit Essigsäureethylester oder anderen nicht mit Wasser mischbaren Lösungsmitteln extrahiert. Aus dem Extrakt werden die Efomycine durch übliche chromatographische Methoden, vorzugsweise Chromatographie an Kieselgel, gewonnen.

Die erfindungsgemäßen Verbindungen zeigen eine gute antibakterielle Wirkung vor allem gegen grampositive Keime. Besonders erwähnt werden soll ihre Eignung zur Verhinderung und Heilung der Schweinedysenterie.

Die Wirkstoffe werden als Leistungsförderer bei Tieren zur Förderung und Beschleunigung des Wachstums, der Milch- und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoff werden bei Nutz-, Zucht-, Zier- und Hobbytieren verwendet.

Zu den Nutz- und Zuchttieren zählten Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Gänse, Enten, Puten, Tauben, Fische wie z.B. Karpfen, Forellen, Lachse, Aale, Schleien, Hechte, Reptilien wie z.B. Schlangen und Krokodile.

Zu den Zier- und Hobbytieren zählen Säugetiere wie Hunde und Katzen, Vögel wie Papageien, Kanarienvögel, Fische wie Zier- und Aquarienfische z.B. Goldfische.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 500 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, der Wachstums- und Leistungsphase, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstums- und Leistungsphase temporär oder kontinuierlich verabreicht werden.

Bei kontinuierlicher Verabreichung kann die Anwendung ein- oder mehrmals täglich in regelmäßigen oder unregelmäßigen Abständen erfolgen.

Die Verabreichung erfolgt oral oder parenteral in dafür geeigneten Formulierungen oder in reiner Form.

Die Wirkstoffe können in den Formulierungen allein oder in Mischung mit anderen leistungsfördernden Wirkstoffen, mineralischen Futtermitteln, Spurenelement-Verbindungen, Vitaminen, Nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind:

z.B. Antibiotika wie Tylosin, Virginiamycin und Monensin. Mineralische Futtermittel sind z.B. Dicalciumphosphat, Magnesiumoxid, Natriumchlorid.

Spurenelement-Verbindungen sind z.B. Eisenfumarat, Natriumjodid, Kobaltchlorid, Kupfersulfat, Zinkoxid.

Vitamine sind z.B. Vitamin A, Vitamin $D_3$, Vitamin E, B-Vitamine, Vitamin C.

Nicht-Protein-Verbindungen sind z.B. Biuret, Harnstoff.

Farbstoffe sind z.B. Carotinoide wie Citranaxanthin, Zeaxanthin, Capsanthin.

Antioxidantien sind z.B. Aethoxyquin, Butylhydroxy-Toluol.

Aromastoffe sind z.B. Vanillin.

8

Emulgatoren sind z.B. Ester der Milchsäure, Lecithin.

Fließhilfsstoffe sind z.B. Natriumstearat, Calciumstearat.

Konservierungsstoffe sind z.B. Zitronensäure, Propionsäure.

Preßhilfsstoffe sind z.B. Ligninsulfonate, Celluloseether.

Die Verabreichung der Wirkstoffe kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Herkunft wie Fleisch, Fette, Milchprodukte, Knochenmehl, Fischprodukte, die Einzelfuttermittel wie Vitamine, Proteine, Aminosäure z.B. DL-Methionin, Salze wie Kalk und Kochsalz. Zum Futter zählen auch Ergänzungs-, Fertig- und Mischfuttermittel. Diese enthalten Einzelfuttermittel in einer Zusammensetzung die eine ausgewogene Ernährung hinsichtlich der Energie- und Proteinversorgung sowie der Versorgung mit Vitaminen, Mineralsalzen und Spurenelementen sicherstellen.

Die Konzentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das erfindungsgemäßen Wirkstoff enthält :

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Prämix ergeben nach sorgfältigem Mischen 1 kg Futter.

In einem kg Futtermischung sind enthalten:

600 I.E. Vitamin A, 100 I.E. Vitamin, $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg Mn $So_2$ x $H_2O$, 140 mg Zn $So_4$ x 7 $H_2O$, 100 mg Fe $SO_4$ x 7 $H_2O$ und 20 mg Cu $So_4$ x 5 $H_2O$.

2,5 g Wirkstoff-Prämix enthalten z.B. 10 mg Wirkstoff, 1 g DL-Methionin, Rest Sojabohnenmehl.

Beispiel für die Zusammensetzung eines Schweineaufzuchtfutters, das erfindungsgemäßen Wirkstoff enthält :

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z.B. wie beim Kükenfutter) ergeben nach sorgfältigem Mischen 1 kg Futter.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

Es ist nicht unbedingt erforderlich, die gereinigten und isolierten Efomycine A-F zu verwenden. Es ist auch möglich, die bei ihrer Herstellung anfallenden Gemische oder sogar die anfallende Kulturbrühe oder das Mycelium ohne Reinigung, gegebenenfalls nach Trocknung, einzusetzen. Für viele Zwecke ist es auch ausreichend, Rohformen der erfindungsgemäßen Wirkstoffe und ihrer Gemische ohne vorherige Feinreinigung einzusetzen.

Die Herstellung und biologische Wirkung der neuen erfindungsgemäßen Verbindungen kann durch die folgenden Beispiele erläutert werden:

Beispiel 1

150 ml steriler, in 1-Ltr.-Erlenmeyerkolben befindlicher Nährlösung (CASO® der Firma Merck, Darmstadt) der Zusammensetzung

```
Pepton aus Casein       15    g
Pepton aus Sojamehl     5     g
Glucose                 2,5   g
NaCl                    5     g
Wasser              ad  1000  ml
```

werden mit vegetativen Zellen des Streptomyceten-Stammes BS 1261 beimpft und 3-4 Tage auf der Rundschüttelmaschine bei 28°C inkubiert. Die gewachsenen Kulturen dienen als Impfgut für weitere Fermentationsansätze.

20 Liter Nährlösung der oben beschriebenen Zusammensetzung und 20 ml Entschäumer (SAG 5693, UnionCarbide) werden in einen Fermenter (30 l) mit Rührwerk und Belüftungseinrichtung abgefüllt, und bei 120°C sterilisiert. Nach Abkühlen der Lösung wird der Fermenter mit dem Inhalt von 2 Kolben der wie oben beschrieben gewonnenen Schüttelkulturen des Streptomyceten-Stammes BS 1261 beimpft, mit 10 Liter steriler Luft pro Minute bei 250 Umdrehungen des Rührwerks pro Minute belüftet und bei einer Temperatur von 28°C und einem Überlagerungsdruck von 0,5 bar fermentiert. Nach 1-2 Tagen wird die Kultivierung beendet und der Inhalt als Inokulum für einen Ansatz in einem 300 l Fermenter verwendet.

Beispiel 2

200 l der Nährlösung der folgenden Zusammensetzung

| | | |
|---|---|---|
| Magermilchpulver | 10 | g |
| Hefeautolysat | 1,5 | g |
| Dextrin | 40 | g |
| D-Glucose | 5 | g |
| Entschäumer | 1 ml | |
| (SAG 5693 Union Carbide) | | |
| ad | 1000 ml $H_2O$ | |

werden in einem 300 l Fermenter auf pH 7 eingestellt, 60 Minuten bei 121°C sterilisiert, auf 28°C abgekühlt und mit 20 l des gemäß Beispiel 1 erhaltenen Inoculums angeimpft. Es wird bei 28°C, einer Belüftungsrate von 100 l steriler Luft pro Minute, bei einem Überlagerungsdruck von 1,0 bar mit 100 Umdrehungen pro Minute für 3-4 Tage gerührt. Dann wird die Kultur geerntet.

Beispiel 3

Die gemäß Beispiel 2 erhaltene Kulturbrühe einer 200-Liter Fermentation wird bei pH7-7,5 mit 200-250 l/h in einem Westfalia-Separator separiert. Das Mycel wird mit dem doppelten Volumen Aceton 30 Minuten bei Raumtemperatur gerührt und zentrifugiert. Der Rückstand wird erneut mit dem doppelten Volumen Aceton gerührt und zentrifugiert. Die vereinigten Zentrifugate werden unter reduziertem Druck bei 40°C Badtemperatur konzentriert. Das Konzentrat wird mit 20 Liter Wasser versetzt und gefriergetrocknet, wobei 540 g des rohen erfindungsgemäßen Efomycin-Gemisches mit einem Gehalt von ca. 5 % an Efomycinen anfallen.

Beispiel 4

60 g des gemäß Beispiel 3 gewonnenen rohen Wirkstoffgemisches werden in 3 Liter Wasser suspendiert und dreimal mit je 400 ml Chloroform extrahiert. Die vereinigten organischen Phasen werden über wasserfreien Natriumsulfat getrocknet, filtriert und unter reduziertem Druck bei 35°C Badtemperatur bis auf ein Volumen von 30 ml eingeengt. Die konzentrierte Lösung läßt man unter Rühren in 2 Liter Heptan einfließen. Nach weiterem Rühren für 30 min wird abfiltriert, wobei 3,6 g Rückstand mit einem Gehalt des Efomycin-Gemisches von ca. 80 % gewonnen werden.

Beispiel 5

3,3 g des nach Beispiel 4 dargestellten Efomycin-Gemisches werden in 10 ml Chloroform gelöst, auf eine in Chlorofrom äquilibrierte Lobar® -Fertigsäule Lichroprep Si 60 (40-63 μm, Merck Größe C) aufgetragen und mit einem Gemisch aus Chloroform und 7 % Methanol fraktioniert eluiert. Die dünnschichtchromatographische Analyse des Eluates (Merck 5715, Chloroform 7 % Methanol, Detektion durch Fluoreszenzlöschung bei 254 nm) ergibt nach fallenden $R_F$-Werten geordnet drei Fraktionen I, II und III, die getrennt im Vakuum bei ca. 35 °C eingedampft werden.

Fraktion I enthält 480 mg eines Produktgemisches, das dickschichtchromatographisch an Kieselgelplatten (Merck 13 895) mit Ethylacetat als Fließmittel in drei Komponenten aufgetrennt wird, die nach steigenden $R_F$-Werten als Efomycin-B (160 mg Ausbeute), Efomycin C (60 mg Ausbeute) bzw. Efomycin D (40 mg Ausbeute) bezeichnet werden.

Fraktion II enthält 950 mg eines Produktgemisches, das dickschichtchromatographisch an Kieselgelplatten (Merck 13 895) mit Ethylacetat als Fließmittel in zwei Komponenten aufgetrennt wird, die nach steigenden $R_F$-Werten als Efomycin A (510 mg Ausbeute) bzw. Efomycin F (320 mg Ausbeute) bezeichnet werden.

Fraktion III enthält als Verdampfungsrückstand 1,6 g Efomycin E, das sich aus Methanol umkristallisieren läßt.

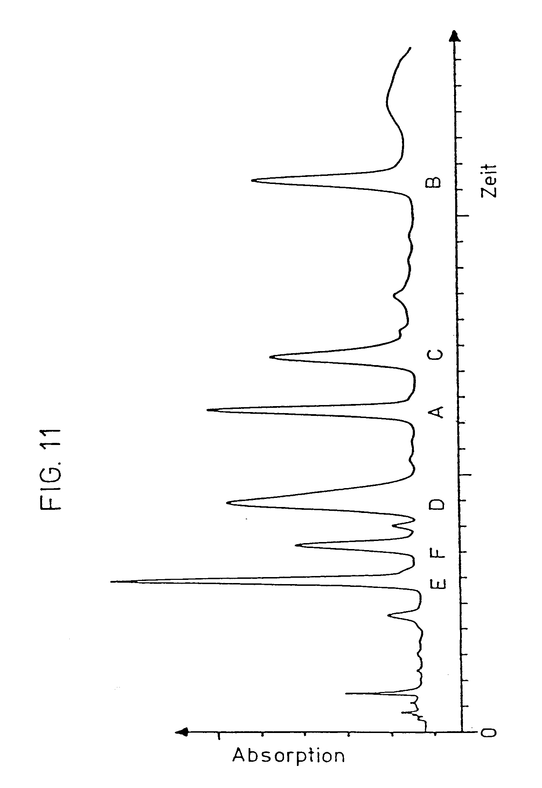

Die Reinheitsgrade der so gewonnenen Efomycin Komponenten A bis E können mittels HPLC (Säule Nova Pak ®, C-18, 18,4 μm, 3,9 x 150 mm; Fa. Waters; Fließmittel: 5 mM Citronensäure mit 45-65 % Acetonitril Zusatz als Gradient in 18 Minuten; Durchflußrate 1,5 ml/min) durch Extinktionsmessungen bei 254 nm und/oder 208 nm Detektorwellenlänge annähernd bestimmt werden. Sie betragen demnach für jede Komponente mindestens 80-85 %. Ein entsprechendes Chromatogramm eines Gemisches der Efomycin-Komponenten A bis E ist in Abb. 11 wiedergegeben.

Beispiel A:

Einem Hammel, der pro Tag 650 g grob gemahlenes Schaf-Fertigfutter und 250 g Trockengrün Cobs erhielt, wurde Pansenflüssigkeit durch eine Pansenfistel entnommen. Das Fertigfutter wurde über einen Futterautomaten in 12 gleichen Portionen in zweistündlichem Abstand und die Cobs in 2 gleichen Portionen um 8.30 und 16.15 Uhr verabreicht. Die Pansenflüssigkeit wurde unmittelbar nach der Gewinnung folgender Behandlung unterworfen: 2,5 ml des Panseninokulums wurden in einem mit Kohlendioxid begasten Reagenzröhrchen von 13 ml Volumen vorgelegt, das darüber hinaus folgende Zusätze enthielt:

| | |
|---|---|
| 100 mg | fein gemahlenes Schaf-Fertigfutter |
| 7,5 ml | Pufferlösung |
| 0,5 ml | einer Lösung mit bzw. ohne erfindungsgemäße Verbindung. |

Die Zusammensetzung der Pufferlösung, welche vor Anfang des Versuches mit Kohlendioxid gesättigt wurde, war wie folgt:

| | | |
|---|---|---|
| $Na_2HPO_4$ | 4,61 g | per Liter Wasser |
| $NaHCO_3$ | 12,25 g | "    "    " |
| $NaCl$ | 0,59 g | "    "    " |
| $KCl$ | 0,71 g | "    "    " |
| $MgCl_2$ | 0,32 g | "    "    " |
| $CaCl_2$ | 0,13 g | "    "    " |

In diese Mischung wurde 0,5 ml einer Lösung, die die gewünschte Menge der erfindungsgemäßen Verbindungen in 5 prozentigem wäßrigem Ethanol enthielt, gegeben. Danach betrug das totale Volumen der Fermentationsmischung 10,5 ml. Jedes Reagenzröhrchen wurde mit einem Bunsen-Stopfen verschlossen und bei 39 °C bebrütet. Nach 2, 4, 6 und 8 Stunden wurden die Ansätze von Hand geschüttelt. Nach 24-stündiger Bebrütung wurde 1,0 ml der Fermentationsflüssigkeit aus den Ansätzen entnommen und in ein Eppendorf-Gefäß pipettiert, das 0,2 ml 10 %ige Phosphorsäure (enthaltend 5,7 μmol 2-Methylvaleriansäure) enthielt. Die Proben wurden bei 11 000 g zentrifugiert und aus dem Überstand die flüchtigen Fettsäurekonzentrationen gaschromatographisch bestimmt.

Es wurde bei jedem Versuch das Verhältnis Essigsäure zu Propionsäure bestimmt. Der bei den Negativkontrollen erhaltene Wert wurde mit 100 angesetzt und die Abweichungen im Verhältnis dazu angegeben. Je mehr Propionsäure gebildet wurde, um so niedriger liegt das Verhältnis Essigsäure zu Propionsäure und um so kleiner wird die Verhältniszahl im Vergleich zur Kontrolle (niedrige Verhältniszahl = verringertes Essigsäure/Propionsäure-Verhältnis = verbesserte Futterverwertung).

Zusätzlich werden bei jedem Versuch die Konzentrationen der Gesamtfettsäuren im Vergleich zur Kontrolle ( = 100) angegeben.

Die Ergebnisse sind in den nachstehenden Tabellen zusammengestellt:

<u>Tabelle a</u>

Gemisch aller Efomycin Komponenten

| Menge (µg/Ansatz) | Essigsäure-Propionsäure-Verhältnis | Gesamtfettsäuren |
|---|---|---|
| Kontrolle | 100 | 100 |
| 20 | 96,7 | 107,1 |
| 50 | 66,9 | 105,4 |
| 100 | 42,5 | 105,8 |
| 250 | 35,6 | 104,9 |
| 500 | 36,7 | 104,8 |

Tabelle b

Efomycin A

| Menge (µg/Ansatz) | Essigsäure- Propionsäure- Verhältnis | Gesamtfettsäuren |
|---|---|---|
| Kontrolle | 100 | 100 |
| 20 | 89,9 | 99,4 |
| 50 | 73,3 | 107,3 |
| 100 | 54,8 | 106,7 |
| 250 | 48,7 | 109,5 |
| 500 | 48,7 | 10,7,2 |

Tabelle c

Efomycin E

| Menge (µg/Ansatz) | Essigsäure- Propionsäure- Verhältnis | Gesamtfettsäuren |
|---|---|---|
| Kontrolle | 100 | 100 |
| 20 | 77,8 | 105,7 |
| 50 | 53,6 | 107,5 |
| 100 | 38,8 | 109,3 |
| 250 | 36,6 | 105,0 |
| 500 | 36,1 | 104,1 |

Beispiel B

Vierundzwanzig Holstein-Friesian Kühe wurden selektiert, um die Effekte der erfindungsgemäßen Verbindungen auf die Milchleistung zu bestimmen. Sechs Kühe bekamen täglich je 1000 mg des Gemisches aller Efomycin Komponenten, sechs bekamen täglich je 1000 mg Efomycin A, sechs bekamen täglich je 1000 mg Efomycin E und sechs Kühe bildeten die Negativkontrollen. Die erfindungsgemäßen Verbindungen wurden in Form eines Prämix dem Fertigfutter der 18 Testkühe zugesetzt. Sämtliche Kühe erhielten eine Ration, die 45 % Mais, 20 % Luzerneheu und 35 % Fertigfutter enthielt. Die Futteraufnahme, Milchproduktion und Milchzusammensetzung (Gehalt an Fett, Eiweiß und Laktose) wurden täglich über 12 Wochen gemessen. Der Versuch fing 4 Wochen nach dem Abkalben an. Bei diesem Versuch wurden gute Ergebnisse erzielt, d.h. eine erhöhte Milchproduktion ohne Beeinflussung der Milchzusammensetzung.

**Patentansprüche**

1. Verwendung der Efomycine A-F oder ihrer Gemische als nichttherapeutische Leistungsförderer bei Tieren, wobei die Efomycine A, B, C, D, F die im folgenden angegebenen chemischen und physikalischen Eigenschaften haben:

a) Efomycin A wird durch die folgenden chemischen und physikalischen Eigenschaften charakterisiert:

1.

| Die Elementaranalyse | C: | 61,3-63,8 |
|---|---|---|
| | H: | 8,2-9,4 |
| | O: | 26,8-30,5 |

2. Summenformel: $C_{55}H_{92}O_{18}$

3. Das Massenspektrum (Fast Atom Bombardment) Molmasse $+ Na^+$: 1061

4. Das $^1$H-Kernresonanzspektrum, angegeben in Teilen pro Million, gemäß Abb. 1

5. Das $^{13}$C-Kernresonanzspektrum, angegeben in Teilen pro Million, gemäß Abb. 2

6. Das UV-Absorptionsmaximum bei 251-254 nm in methanolischer Lösung.

b) Efomycin B wird durch die folgenden chemischen und physikalischen Eigenschaften charakterisiert:

1.

| Die Elementaranalyse | C: | 61,3-63,8 |
|---|---|---|
| | H: | 8,2-9,4 |
| | O: | 26,8-30,5 |

2. Summenformel: $C_{56}H_{94}O_{18}$

3. Das Massenspektrum (Fast Atom Bombardment) Molmasse $+ Na^+$: 1075

4. Das $^1$H-Kernresonanzspektrum, angegeben in Teilen pro Million, gemäß Abb. 3.

5. Das $^{13}$C-Kernresonanzspektrum, angegeben in Teilen pro Million, gemäß Abb. 4.

6. Das UV-Absorptionsmaximum bei 251-254 nm in methanolischer Lösung.

c) Efomycin C wird durch die folgenden chemischen Eigenschaften charakterisiert.

1. Die Summenformel: $C_{49}H_{80}O_{15}$

2. Das Massenspektrum (Fast Atom Bombardment) Molmasse $+ Na^+$: 931

3. Das $^1$H-Kernresonanzspektrum, angegeben in Teilen pro Million, gemäß Abb. 5.

4. Das $^{13}$C-Kernresonanzspektrum, angegeben in Teilen pro Million, gemäß Abb. 6.

5. Das UV-Absorptionsmaximum bei 251-254 nm in methanolischer Lösung.

d) Efomycin D wird durch die folgenden chemischen und physikalischen Eigenschaften charakterisiert:

1. Die Summenformel: $C_{42}H_{68}O_{12}$

2. Das Massenspektrum (Fast Atom Bombardment) Molmasse $+ Na^+$: 787

3. Das $^1$H-Kernresonanzspektrum, angegeben in Teilen pro Million (Abb. 7) $\mu$mol (Abb. 8).

4. Das $^{13}$C-Kernresonanzspektrum, angegeben in Teilen pro Million, gemäß Abb. 9.

5. Das UV-Absorptionsmaximum bei 251-254 nm in methanolischer Lösung.

e) Efomycin F wird durch die folgenden chemischen und physikalischen Eigenschaften charakterisiert:

1. Die Summenformel $C_{48}H_{78}O_{15}$

2.

| Die Elementaranalyse | C: | 63,0-65,6 |
|---|---|---|
| | H: | 8,5-8,9 |
| | O: | 26,5-28,5 |

14

3. Das Massenspektrum (Fast Atom Bombardment) Molmasse $+ \text{Na}^{+}$: 917

4. Das UV-Absorptionsmaximum bei 251-254 nm in methanolischer Lösung.

5. Die folgenden Absorptionsbanden, angegeben in $\text{cm}^{-1}$, des IR-Spektrums, aufgenommen als KBr Preßling:

| | | | |
|------|------|------|-----|
| 3444 | 1385 | 1150 | 745 |
| 2975 | 1304 | 1112 | |
| 2937 | 1284 | 1088 | |
| 1693 | 1259 | 1000 | |
| 1642 | 1226 | 880 | |
| 1462 | 1187 | 825 | |

2. Mittel zur Leistungsförderung von Tieren gekennzeichnet durch einen Gehalt an mindestens einem Efomycin A-D + F oder an Gemischen derselben.

3. Tierfutter, Trinkwasser für Tiere, Zusätze für Tierfutter und Trinkwasser für Tiere gekennzeichnet durch einen Gehalt an mindestens einem Efomycin A-D + F oder an Gemischen derselben.

4. Efomycine A, B, C, D, F mit den in Anspruch 1 angegebenen chemischen und physikalischen Eigenschaften sowie Gemische der Efomycine A, B, C, D, F.

**Claims**

1. Use of efomycins A-F or their mixtures as non-therapeutic yield promoters in animals, where efomycins A, B, C, D and F have the chemical and physical properties given below:

   a) Efomycin A is characterised by the following chemical and physical properties:
   1.

| The elemental analysis | C: | 61.3-63.8 |
|------------------------|-----|-----------|
| | H: | 8.2- 9.4 |
| | O: | 26.8-30.5 |

   2. Empirical formula: $C_{55}H_{92}O_{18}$
   3. The mass spectrum (fast atom bombardment) molar mass $+ \text{Na}^{+}$: 1,061
   4. The $^{1}$H-nuclear magnetic resonance spectrum, given in parts per million, according to Figure 1
   5. The $^{13}$C-nuclear magnetic resonance spectrum, given in parts per million, according to Figure 2
   6. The UV absorption maximum at 251-254 nm in methanolic solution.

   b) Efomycin B is characterised by the following chemical and physical properties:
   1.

| The elemental analysis | C: | 61.3-63.8 |
|------------------------|-----|-----------|
| | H: | 8.2- 9.4 |
| | O: | 26.8-30.5 |

   2. Empirical formula: $C_{56}H_{94}O_{18}$
   3. The mass spectrum (fast atom bombardment) molar mass $+ \text{Na}^{+}$: 1,075
   4. The $^{1}$H-nuclear magnetic resonance spectrum, given in parts per million, according to Figure 3.
   5. The $^{13}$C-nuclear magnetic resonance spectrum, given in parts per million, according to Figure 4.
   6. The UV absorption maximum at 251-254 pm in methanolic solution.

   c) Efomycin C is characterised by the following chemical properties:
   1. The empirical formula $C_{49}H_{80}O_{15}$
   2. The mass spectrum (fast atom bombardment) molar mass $+ \text{Na}^{+}$: 931
   3. The $^{1}$H-nuclear magnetic resonance spectrum, given in parts per million, according to Figure 5.

4. The $^{13}$C-nuclear magnetic resonance spectrum, given in parts per million, according to Figure 6.

5. The UV absorption maximum at 251-254 nm in methanolic solution.

d) Efomycin D is characterised by the following chemical and physical properties:

1. The empirical formula: $C_{42}H_{68}O_{12}$

2. The mass spectrum (fast atom bombardment) molar mass + Na$^+$: 787

3. The $^1$H-nuclear magnetic resonance spectrum, given in parts per million (Figure 7) and (Figure 8).

4. The $^{13}$C-nuclear magnetic resonance spectrum, given in parts per million, according to Figure 9.

5. The UV absorption maximum at 251-254 nm in methanolic solution.

e) Efomycin F is characterised by the following chemical and physical properties:

1. The empirical formula: $C_{48}H_{78}O_{15}$

2.

| The elemental analysis | C: | 63.0-65.6 |
|---|---|---|
| | H: | 8.5- 8.9 |
| | O: | 26.5-28.5 |

3. The mass spectrum (fast atom bombardment) molar mass + Na$^+$: 917

4. The UV absorption maximum at 251-254 nm in methanolic solution.

5. The following absorption bands, given in cm$^{-1}$, of the IR spectrum, recorded as a KBr disc:

| | | | |
|---|---|---|---|
| 3444 | 1385 | 1150 | 745. |
| 2975 | 1304 | 1112 | |
| 2937 | 1284 | 1088 | |
| 1693 | 1259 | 1000 | |
| 1642 | 1226 | 880 | |
| 1462 | 1187 | 825 | |

2. Agents for promoting the yield of animals, characterised in that they contain at least one efomycin A-D + F or mixtures thereof.

3. Animal feed, drinking water for animals, additives for animal feed and drinking water for animals, characterised in that they contain at least one efomycin A-D + F or mixtures thereof.

4. Efomycins A, B, C, D and F with the chemical and physical properties given in Claim 1, and mixtures of efomycins A, B, C, D and F.

**Revendications**

1. Utilisation des éfomycines A-F ou de leurs mélanges comme substances non thérapeutiques augmentant le rendement chez les animaux, les éfomycines A, B, C, D, F ayant les propriétés chimiques et physiques indiquées ci-après :

a) l'éfomycine A est caractérisée par les propriétés chimiques et physiques suivantes :

1.

| Analyse élémentaire | C: | 61,3-63,8 |
|---|---|---|
| | H: | 8,2-9,4 |
| | O: | 26,8-30,5 |

2. Formule brute : $C_{55}H_{92}O_{18}$

3. Spectre de masse (bombardement par atomes rapides)

Masse moléculaire + Na$^+$:1061

4. Spectre de résonance des noyaux de $^1H$, indiqué en parties par million, conforme à la figure 1.

5. Spectre de résonance des noyaux de $^{13}C$, indiqué en parties par million, conforme à la figure 2.

6. Maximum d'absorption de la lumière ultra-violette à 251-254 nm en solution méthanolique.

b) L'éfomycine B est caractérisée par les propriétés chimiques et physiques suivantes :

1.

| Analyse élémentaire | C: | 61,3-63,8 |
|---|---|---|
| | H: | 8,2-9,4 |
| | O: | 26,8-30,5 |

2. Formule brute : $C_{56}H_{94}O_{18}$

3. Spectre de masse (bombardement par atomes rapides)

Masse moléculaire $+Na^+$ :1075

4. Spectre de résonance des noyaux de $^1H$, indiqué en parties par million, conforme à la figure 3.

5. Spectre de résonance des noyaux de $^{13}C$, indiqué en parties par million, conforme à la figure 4.

6. Maximum d'absorption de la lumière ultra-violette à 251-254 nm en solution méthanolique.

c) L'éfomycine C est caractérisée par les propriétés chimiques suivantes :

1. Formule brute : $C_{49}H_{80}O_{15}$

2. Spectre de masse (bombardement par atomes rapides)

Masse moléculaire $+Na^+$ :931

3. Spectre de résonance des noyaux de $^1H$, indiqué en parties par million, conforme à la figure 5.

4. Spectre de résonance des noyaux de $^{13}C$, indiqué en parties par million, conforme à la figure 6.

5. Maximum d'absorption de lumière ultraviolette à 251-254 nm en solution méthanolique.

d) L'éfomycine D est caractérisée par les formules chimiques et physiques suivantes :

1. Formule brute : $C_{42}H_{68}O_{12}$

2. Spectre de masse (bombardement par atomes rapides) Masse moléculaire $+Na^+$ :787

3. Spectre de résonance des noyaux de $^1H$, indiqué en parties par million (figure 7) et (figure 8).

4. Spectre de résonance des noyaux de $^{13}C$, indiqué en parties par million, conforme à la figure 9.

5. Maximum d'absorption de lumière ultraviolette à 251-254 nm en solution méthanolique.

e) L'éfomycine F est caractérisée par les propriétés chimiques et physiques suivantes :

1. Formule brute : $C_{48}H_{78}O_{15}$

2.

| Analyse élémentaire | C: | 63,0-65,6 |
|---|---|---|
| | H: | 8,5- 8,9 |
| | O: | 26,5-28,5 |

3. Spectre de masse (bombardement par atomes rapides)

Masse moléculaire $+Na^+$ :917

4. Maximum d'absorption de la lumière ultra-violette à 251-254 nm en solution méthanolique.

5. Bandes d'absorption suivantes, indiquées en $cm^{-1}$, du spectre infrarouge, mesuré sur pastilles de KBr :

| 3444 | 1385 | 1150 | 745 |
|---|---|---|---|
| 2975 | 1304 | 1112 | |
| 2937 | 1284 | 1088 | |
| 1693 | 1259 | 1000 | |
| 1642 | 1226 | 880 | |
| 1462 | 1187 | 825 | |

17

2. Composition destinée à augmenter le rendement chez les animaux, caractérisée par une teneur en au moins une éfomycine A-D + F ou en mélanges de ces éfomycines.

3. Aliment pour animaux, eau de boisson pour animaux, additifs pour aliments et eau de boisson pour animaux, caractérisés par une teneur en au moins une éfomycine A-D + F ou en mélanges de ces éfomycines.

4. Efomycines A, B, C, D, F possédant les propriétés chimiques et physiques indiquées dans la revendication 1, ainsi que des mélanges des éfomycines A,B,C,D,F.

FIG. 1

⊗ Solvent

# FIG. 2

⊗ Solvent

200   180   160   140   120   100   80   60   40   20   0 ppm

FIG. 3

⊗ Solvent

EP 0 197 360 B1

# FIG. 4

⊗ Solvent

△ TMS

200  180  160  140  120  100  80  60  40  20  0  ppm

EP 0 197 360 B1

# FIG. 5

⊗ Solvent

△ TMS

EP 0 197 360 B1

# FIG. 6

⊗ Solvent
□ Impurity

EP 0 197 360 B1

# FIG. 7

⊗ Solvent
△ TMS

EP 0 197 360 B1

FIG. 8

⊗ Solvent

△ TMS

FIG. 9

⊗ Solvent
□ Impurity

Efomycin E

FIG. 10

FIG. 11